# FASCICULE DE BREVET EUROPEEN

(11) **EP 1 888 140 B1**
(45) Date de publication et mention de la délivrance du brevet: **03.12.2008**
(21) Numéro de dépôt: 05777325.1
(22) Date de dépôt: 09.06.2005
(51) Int. Cl.: A61M 1/00, A61M 3/02, A61B 1/12

(54) **DISPOSITIF D IRRIGATION ET DE MISE EN PRESSION D UNE CAVITE**
VORRICHTUNG ZUR IRRIGATION UND DRUCKBEAUFSCHLAGUNG EINES HOHLRAUMS
DEVICE FOR IRRIGATING AND PRESSURIZING A CAVITY

(43) Date de publication de la demande: 20.02.2008
(73) Titulaire: Guignard, Mireille, F-01630 Sergy (FR)
(72) Inventeur: Guignard, Mireille, F-01630 Sergy (FR)
(74) Mandataire: Savoye, Jean-Paul
(86) Numéro de dépôt international: PCT/FR2005/001435
(87) Numéro de publication internationale: WO 2006/131609

(56) Documents cités:
- FR-A- 2 766 083
- US-A- 4 696 669
- US-A- 4 902 276
- US-A- 4 998 914
- US-A- 5 195 960

## Description

La présente invention a pour objet un dispositif d'irrigation et de mise en pression d'une cavité et en particulier un dispositif permettant de maintenir constante la pression exercée par un fluide au sein de cette cavité, quelles que soient les variations de débit de ce fluide.

Dans le domaine médical, il est connu d'utiliser des systèmes permettant de faire circuler un fluide, liquide ou gazeux, au travers d'une cavité humaine ou animale en vue d'y pratiquer une intervention endoscopique. Typiquement, une telle intervention peut servir à l'examen ou au prélèvement des tissus de la cavité, par exemple en vue d'établir un diagnostique ultérieur ou d'opérer par résection l'intérieur de cette cavité pour y retirer une tumeur.

Afin de maintenir un espace suffisant à la manipulation des instruments utilisés lors de l'opération, il convient de mettre la cavité sous pression pour augmenter légèrement son volume, du moins pour s'assurer qu'elle ne se rétracte pas.

La mise sous pression de la cavité est généralement réalisée par le biais d'une alimentation en sérum physiologique et/ou d'une insufflation d'un gaz dans cette cavité. L'irrigation de cette cavité a également pour rôle d'évacuer, lors d'une opération de rinçage, les matières organiques provenant d'une résection des tissus. Sans une telle irrigation ces déchets organiques opacifieraient rapidement l'image du champ opératoire donnée par l'endoscope. En débouchant dans la cavité, le tube opérateur de l'endoscope sert donc également de conduit d'alimentation pour le sérum physiologique, alors qu'un conduit d'évacuation permet d'acheminer le sérum souillé vers l'extérieur avant de le collecter dans un récipient.

Le débit de la circulation du sérum peut être régulé par un simple robinet, plus généralement par une pompe péristaltique. Fonctionnant par compression du conduit d'évacuation contre le corps de pompe lors de la rotation de patins solidaires du rotor, une telle pompe permet avantageusement de garantir une totale stérilité du fait qu'aucune partie mécanique ne vient en contact avec le liquide aspiré.

La pression de consigne dans le circuit d'alimentation varie essentiellement en fonction des tissus qui forment la cavité opérée. Le maintien de cette pression à une valeur constante est de première importance. En effet, une surpression pourrait provoquer des lésions à la cavité elle-même, voire des infiltrations de liquide dans le corps du patient. A l'inverse, une diminution de la pression pourrait détendre les parois de la cavité, les mettre en contact avec l'instrumentation introduit dans la cavité et engendrer une perforation accidentelle de celle-ci accompagnée de graves conséquences.

Il est connu d'ajuster, par gravité, la pression au sein de la cavité en faisant varier la hauteur de la colonne de sérum située dans le conduit d'alimentation. Cela revient, en d'autres termes, à faire varier la hauteur du réservoir de liquide physiologique par rapport au niveau de la cavité opérée.

Plus perfectionné, le document EP 701'456 divulgue un dispositif pour alimenter en liquide et mettre une cavité du corps humain ou d'un animal sous une pression déterminée. A cet effet, une poche souple remplie de liquide physiologique est agencée dans une enceinte étanche. Cette dernière est mise sous pression par une source d'air pressurisée qui est connectée à un régulateur de pression ainsi qu'à un indicateur de pression. La régulation du débit du liquide physiologique, ainsi mis sous pression, est contrôlée en aval de la cavité par une pompe péristaltique agissant sur le conduit d'évacuation.

L'inconvénient de ce dispositif réside dans le fait que, lorsque le praticien commande un rinçage de la cavité, par augmentation significative du débit, une dépression se crée dans la cavité suite à l'augmentation immédiate de la vitesse de rotation de la pompe. Cette baisse de pression est essentiellement provoquée par la hausse soudaine des pertes de charge résultant de l'augmentation de la vitesse du fluide dans le conduit amont, à savoir celui situé entre la poche-réservoir de liquide et la cavité. Le temps de réponse à la compensation de ces pertes de charge n'étant pas nul, la pression dans la cavité diminue quelque peu avant de revenir à sa valeur nominale lorsque le système hydraulique se stabilise à la vitesse de rinçage. De ces variations de pression, il en résulte un effet pulsant indésirable des parois de la cavité, pouvant conduire à de sérieuses conséquences en cas de perforation comme évoqué plus haut.

Dans le même domaine, le document FR 2'766'083 divulgue un dispositif permettant de réaliser un procédé de détermination de pertes de liquide dans le circuit d'irrigation d'une cavité humaine ou animale. Pour ce faire le circuit comprend un conduit d'alimentation reliant un réservoir de liquide à la cavité, ainsi qu'un conduit d'évacuation dans lequel le liquide souillé est évacué de la cavité jusqu'à un réservoir collecteur. Deux pompes péristaltiques agencées sur ce circuit permettent la circulation du liquide. L'une de ces pompes est située en amont sur le conduit d'alimentation et l'autre en aval sur le conduit d'évacuation. Un capteur de pression est disposé sur le conduit d'alimentation, entre la première pompe et la cavité. Ce capteur est destiné à mesurer la pression dans le circuit délimité par les deux pompes et à détecter, via une unité centrale de commande, une éventuelle baisse de pression significative due à une fuite dans ce circuit. Pour ce faire, la méthode prévoit de stopper fréquemment les deux pompes pour pouvoir mesurer la différence de pression existant entre le début et la fin de l'arrêt des pompes.

Ce dispositif nécessite, dans le circuit hydraulique, l'agencement d'un capteur de pression accouplé à un réservoir supplémentaire. Un tel capteur mesure la pression du volume d'air surmontant la surface du liquide dans ce réservoir. Cet air fait également office d'amortisseur pour éviter le phénomène de coups de bélier provoquant des à-coups néfastes à la pompe située en amont. Cependant, un tel amortisseur à air ne fait qu'atténuer la transition d'une valeur de pression à une autre mais ne permet ni de réduire son amplitude, ni de supprimer toute variation de pression lors du rinçage.

En outre, le capteur de pression de ce dispositif requiert l'agencement d'un filtre micro-poreux stérile permettant d'éviter tout contact entre le liquide et le capteur. Or, les inconvénients que procure l'agencement d'un tel filtre résident dans le fait qu'un contact accidentel entre le liquide et le filtre peut fausser la mesure de pression, voire plus gravement obstruer totalement les pores du filtre et isoler ainsi le capteur de pression de toute variation possible. Cette méthode présente encore l'inconvénient de nécessiter l'arrêt des pompes pour pouvoir détecter puis parer à une éventuelle baisse de pression.

Le but de la présente invention vise à remédier, au moins en partie, aux inconvénients précités en suggérant un dispositif permettant la régulation d'un fluide dans une cavité en y maintenant la pression constante malgré des variations momentanées de débit.

A ces fins, la présente invention a pour objet un dispositif conforme à ce qu'énonce la revendication 1.

Les principaux avantages de ce dispositif résident dans le fait qu'il permet de s'affranchir de l'effet pulsant des parois de la cavité engendré par des variations de débit, en garantissant une stabilité de la pression au sein de cette cavité. En outre, il présente l'avantage d'être de conception simple, fiable et économique, tout en écartant tout danger pour le patient en cas de fuite ou de disfonctionnement éventuel d'un des organes de ce dispositif.

D'autres avantages apparaîtront à la lumière de la description qui va suivre et qui se réfère à un mode de réalisation préféré, pris à titre nullement limitatif, et illustré par les figures annexées dans lesquelles:
La figure 1 est une vue schématique du dispositif objet de la présente invention illustré dans un premier état.
La figure 2 représente une portion du dispositif illustré à la figure 1, suivant un deuxième état.
Les figure 3a, 3b et 3c sont des illustrations des différents états possibles, représentés chacun dans une vue en coupe verticale simplifiée de la figure 2 à l'axe de rotation de la pompe.
La figure 4 illustre une première variante du dispositif tel que schématisé à la figure 1.
La figure 5 représente, dans un schéma de principe, une seconde variante du dispositif objet de la présente invention.

Afin d'éviter toute ambiguïté, on définira les termes amont et aval comme faisant référence au sens d'écoulement du fluide dans les conduits du dispositif de la présente invention.

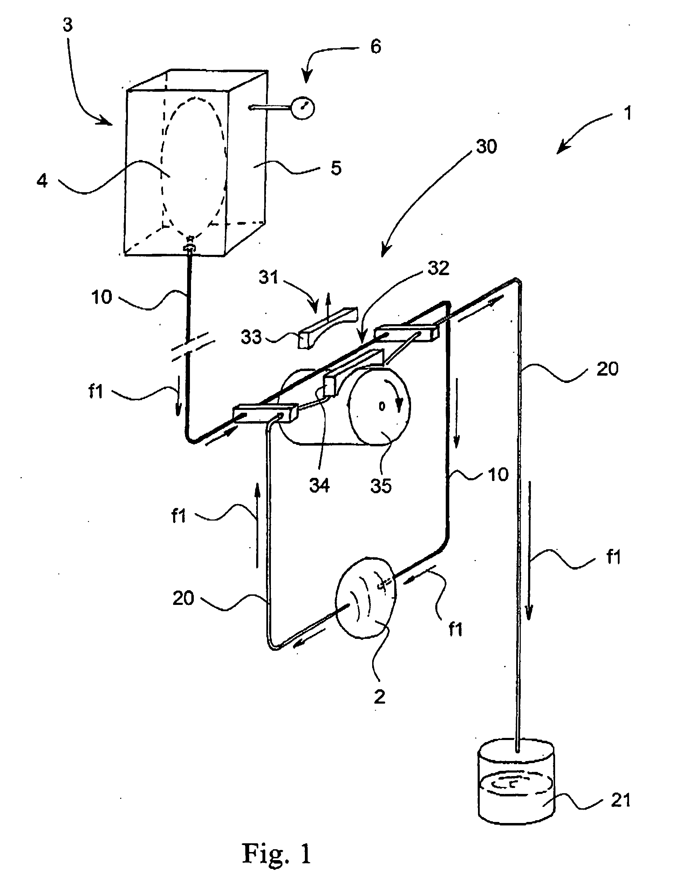

En référence à la figure 1, celle-ci illustre schématiquement le dispositif 1 d'irrigation et de mise en pression d'une cavité 2 organique, tel qu'une cavité du corps humain ou d'un animal. Issu d'une source 3, un fluide sous pression circule dans un circuit de conduits conformément aux flèches f1 représentées le long de ces derniers. Ces conduits sont typiquement réalisés à partir d'un matériau souple et sont généralement de faible diamètre. Par conséquent, les pertes de charge propres à ces tuyaux peuvent devenir relativement importantes lorsqu'ils sont pincés, lorsqu'ils forment des coudes ou lorsque le débit d'écoulement augmente sensiblement. La mise en pression du fluide peut être obtenue d'une façon quelconque, par exemple en employant une poche souple faisant office de réservoir 4 placé dans une enceinte étanche 5, contrôlée et mise sous pression à une valeur de consigne au moyen d'un régulateur 6.

De la source 3 est issu un conduit d'alimentation 10 débouchant en son extrémité aval dans la cavité 2. Après avoir rempli et traversé cette cavité, le fluide ressort de cette dernière par un conduit d'évacuation 20 pour être finalement collecté dans un récipient 21.

Des moyens de régulation 30 de ce fluide permettent de contrôler son débit dans le circuit formé par le conduit d'alimentation 10 et le conduit d'évacuation. 20. D'autre part, ces moyens de régulation permettent également de maintenir constante la pression au sein de la cavité, à savoir de la maintenir à la valeur de consigne.

Pour ce faire, ces moyens de régulation comprennent un premier moyen de pompage 31 pouvant être momentanément en prise avec le conduit d'alimentation 10, ainsi qu'un second moyen de pompage 32 en prise avec le conduit d'évacuation 20. Par l'expression momentanément en prise, on entend que le premier moyen de pompage peut être temporairement accouplé ou embrayé avec le conduit auquel il est normalement dissocié la majeure partie du temps.

En référence à la figure 1, le premier moyen de pompage 31 est réalisé par l'association d'un rotor de pompe péristaltique 35 et d'un premier organe mobile entre deux positions principales. Un tel organe est schématisé dans cette figure par un premier sabot 33, rétractable du conduit d'alimentation 10. La première position de ce sabot est celle dans laquelle il est en prise avec le conduit d'alimentation, alors que la seconde position est celle dans laquelle il est en retrait de ce conduit. Le second moyen de pompage 32 est de préférence réalisé par l'association du même rotor de pompe péristaltique 35 et d'un second organe mobile, semblable ou identique au premier, tel qu'un second sabot 34. De préférence, le second sabot 34 est destiné à rester en prise avec le conduit qui lui est associé, à savoir avec le conduit d'évacuation 20.

Tel que représenté en figure 1, le premier moyen de pompage 31 et le second moyen de pompage 32 constituent ensemble une seule pompe péristaltique, de type particulier, que l'on qualifiera de pompe péristaltique à double conduits ou à double flux. De par le premier sabot 33, voire le second sabot 34, cette pompe est équipée d'au moins un moyen permettant de neutraliser l'effet de pompage sur au moins un des conduits 10, 20.

Le fonctionnement du dispositif 1 de la présente invention va maintenant pouvoir être expliqué avec l'appui des figures 1 à 3c. Lorsque aucun rinçage de la cavité n'est ordonné, la configuration du dispositif est celle illustrée à la figure 1. Dans ce premier état, le premier moyen de pompage 31 se trouve en retrait du conduit d'alimentation 10, lequel conduit est ainsi libre de toute contrainte. La pression régnant au sein du circuit, cavité 2 comprise, est engendrée par le fluide, lequel est mis sous pression par la source 3 à une valeur de consigne constante. Du fait que la source 3 soit généralement pourvue d'un système de sécurité, non représenté, aucune surpression au-delà de la valeur de consigne ne peut être engendrée par erreur. Ce premier état correspondant à la situation dans laquelle le praticien opère ou effectue un diagnostique au sein de la cavité 2, irriguée par un fluide tel que du sérum physiologique s'écoulant à un débit faible et constant.

Lorsque le fluide emplissant la cavité devient trop trouble pour poursuivre l'intervention dans de bonnes conditions, le praticien va actionner une commande qui entraînera l'opération de rinçage de la cavité. Cette opération se traduit par une augmentation significative du débit du fluide, emportant ainsi tous les déchets organiques s'y trouvant en sustentation vers le récipient 21, via le conduit d'évacuation 20. Le rinçage de la cavité correspond donc à une opération non seulement marginale mais également très limitée dans le temps.

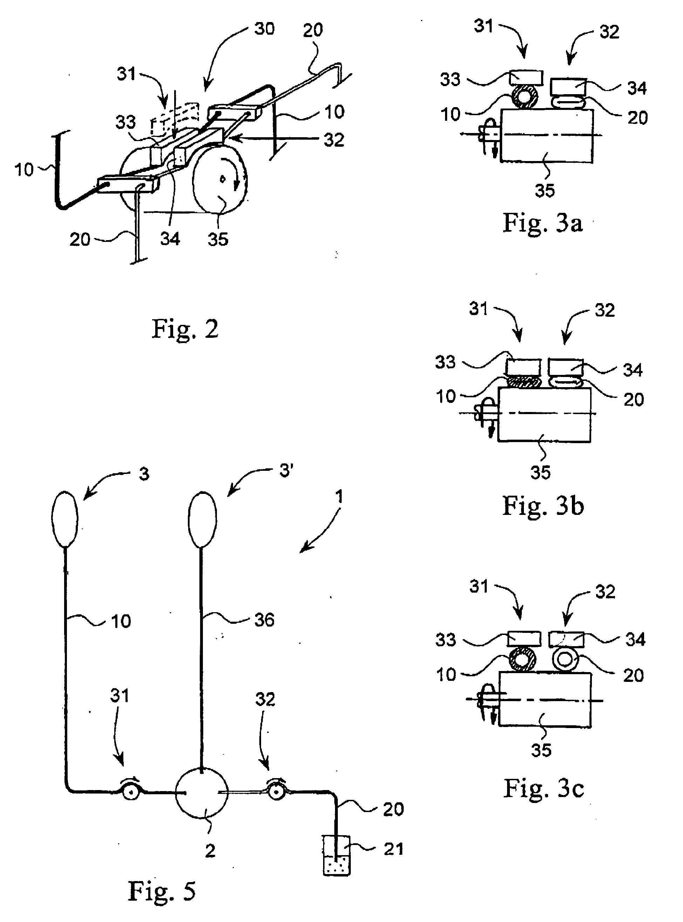

Lors de chaque rinçage, la forte augmentation du débit du fluide provoque une hausse substantielle des pertes de charge dans le circuit du dispositif 1. Pour éviter toute diminution de la pression dans la cavité due aux variations subites de l'importance de ces pertes de charge, l'opération de rinçage est précédée par l'actionnement du premier moyen de pompage 31. Tel qu'illustré à la figure 2, cet actionnement se concrétise par la fermeture du premier sabot 33 jusqu'à ce que ce dernier vienne en prise avec le conduit d'alimentation 10. Cette fermeture provoque l'écrasement et le maintient du conduit d'alimentation contre le rotor de la pompe péristaltique, plus précisément contre les galets agencés sur ce rotor. De ce second état, il en résulte une fermeture momentanée du tronçon englobant la cavité 2, délimité en amont par le premier moyen de pompage 31 et en aval par le second moyen de pompage 32. En terme de pression, ce tronçon se trouve comme isolé du reste du circuit et de ce fait se trouve à l'abri de toute variation de pression engendrée par les variations de pertes de charge lors des modifications de débits. Une fois le rinçage terminé, le débit du fluide revient à sa valeur initial, après quoi le premier moyen de pompage 31 est désactivé, neutralisant ainsi l'effet de pompage sur le conduit d'alimentation.

La figure 3a illustre, dans une coupe partielle verticale de la figure 2, le premier état dans lequel seul l'effet de pompage est appliqué au conduit d'évacuation 20 par l'action du second moyen de pompage 32. Dans la figure 2, ce premier état est représenté en trait interrompu par la position rétractée du premier sabot 33.

La figure 3b illustre, dans une coupe semblable à celle de la figure 3a, le second état dans lequel l'effet de pompage est appliqué simultanément aux deux conduits 10, 20, par l'action des deux moyens de pompage 31, 32 correspondants.

La figure 3c représente une troisième situation dans laquelle l'irrigation de la cavité est rendue libre par neutralisation simultanée de l'effet de pompage sur les deux conduits 10 et 20. Une telle situation pourrait se présenter dans le cas où le second moyen de pompage 32 pourrait également être constitué d'un moyen débrayable par rapport au conduit qui lui est associé. Il en résulterait un possible désaccouplement de ces deux derniers organes.

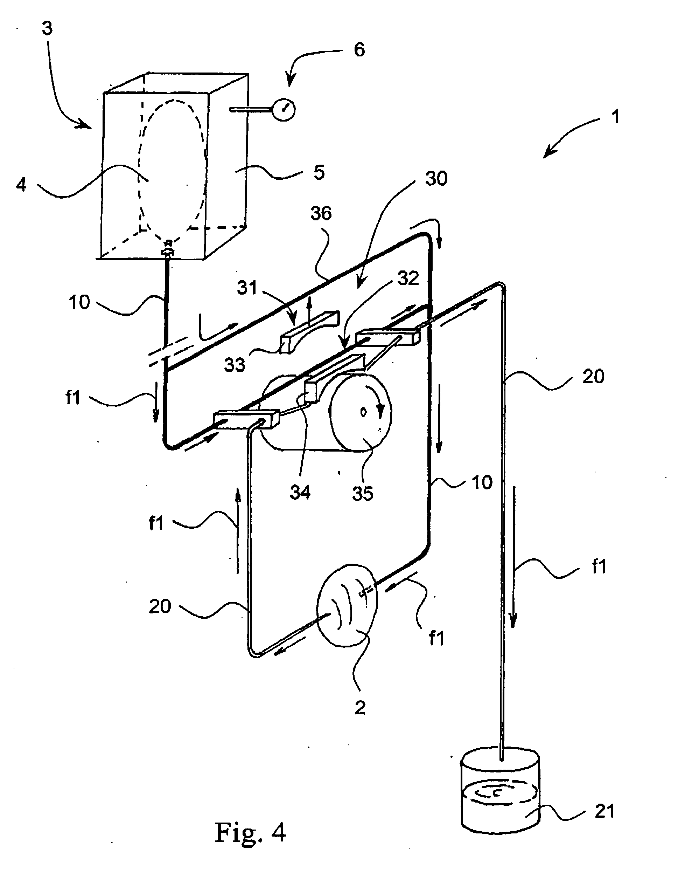

La figure 4 illustre une première variante du dispositif tel que représenté à la figure 1, dans laquelle les moyens de régulation 30 comprennent également un conduit de dérivation 36 permettant de mettre la cavité 2 directement sous pression, à la même valeur que celle du fluide. Pour ce faire, l'extrémité amont du conduit de dérivation 36 est de préférence connectée à la sortie de la source 3 et l'extrémité aval de ce conduit est de préférence connectée au conduit d'alimentation, entre le premier moyen de pompage 31 et la cavité 2. Grâce à ce conduit de dérivation additionnel, la cavité peut être directement mise sous pression, à la valeur de consigne, sans passer par le premier moyen de pompage 31. En cas de panne de ce dernier, la pression au sein de la cavité peut être maintenue à la valeur de consigne, évitant ainsi tout risque de dégonflement de cette cavité.

En outre, l'utilisation d'un tel conduit de dérivation permet avantageusement de dissocier les deux grandeurs physiques que sont la pression et le débit dans le circuit du dispositif 1. En effet, grâce à ce conduit de dérivation 40, il devient possible d'appliquer une certaine pression de consigne au sein de la cavité 2 tout en choisissant de manière indépendante le débit du fluide s'écoulant dans cette cavité par modification de la vitesse des autres moyens de régulation 30, à savoir du premier moyen de pompage 31 et du second moyen de pompage 32.

La figure 5 représente, dans un schéma de principe, une seconde variante du dispositif objet de la présente invention dans laquelle on remarque que les moyens de régulation 30 sont au moins constitués des deux moyens de pompage 31, 32, séparés l'un de l'autre. Ainsi, le premier moyen de pompage 31 est constitué par une pompe péristaltique pourvue d'un moyen 33 permettant de neutraliser l'effet de pompage sur le conduit d'alimentation et le second moyen de pompage 32 est constitué par une seconde pompe péristaltique, semblable à la première. Bien qu'étant physiquement séparées, les deux pompes sont reliées l'une à l'autre, soit mécaniquement, soit par un arbre électrique. Lorsqu'elles sont en prise avec leurs conduits respectifs, ces pompes peuvent ainsi être activées à la même vitesse, dans le même sens et de préférence en phase l'une avec l'autre. Dans cette seconde variante, le conduit de dérivation 36 est issu d'une seconde source 3' mise sous pression et connectée directement à la cavité 2. On comprendra que ce schéma illustre une variante plus théorique que pratique dans laquelle les fluides des deux sources 3, 3' sont d'une part identiques et d'autre part soumis à une pression égale. Par conséquent, on comprendra également que le conduit de dérivation 36 pourrait être issu d'un point quelconque du conduit d'alimentation 10 situé entre la source 3 et le premier moyen de pompage 31. De même, l'extrémité aval de ce conduit de dérivation pourrait être reliée en un point quelconque du conduit d'alimentation situé entre le premier moyen de pompage 31 et la cavité 2.

En référence à la figure 5, l'agencement du conduit de dérivation 36 au sein du dispositif 1 pourvu de deux moyens de pompage 31, 32 physiquement séparés, permet d'écarter tout danger pour le patient en cas de panne de l'un ou l'autre de ces moyens de pompage. En effet, dans le cas où le premier moyen de pompage 31 serait mis hors d'usage, la mise en pression et l'irrigation de la cavité 2 serait maintenue par la voie du conduit 36 et le fonctionnement du second moyen de pompage 32. A l'inverse, si le second moyen de pompage 32 tombait en panne, la pression et l'irrigation de la cavité 2 serait avantageusement maintenue par un circuit en boucle formé du conduit d'alimentation 10, du premier moyen de pompage 31 et du conduit de dérivation 36.

Grâce à la pompe à double flux schématisée aux figures 1 à 4, tout défaut de synchronisation des deux moyens de pompage 31, 32 peut avantageusement être écarté. De par sa conception, une telle pompe permet en effet de garantir un fonctionnement en phase et à la même vitesse des deux moyens de pompage lorsque ces derniers sont tous deux en action via leurs sabots 33, 34 respectifs. Avantageusement encore, ce mode de réalisation permet d'éviter tout risque d'accident en cas de disfonctionnement de la pompe. Du fait que le rotor de la pompe péristaltique 35 permette le fonctionnement simultané des deux moyens de pompage 31, 32, toute panne de ce rotor mettra également ces deux moyens de pompage hors fonction de manière conjointe. De ce fait, le risque de voir la cavité 2 se vider malencontreusement de son fluide par la seule action du second moyen de pompage 32, suite à une panne du premier, est totalement écarté. Il en va de même du risque de voir cette cavité se gonfler de façon excessive par la seule action du premier moyen de pompage 31, par suite d'une panne du second. En outre, en cas de panne de la pompe péristaltique 35 à double flux, la pression au sein de la cavité est avantageusement maintenue par le conduit de dérivation 36. Comme bien illustré à la figure 5, tout risque de refoulement du fluide dans ce conduit de dérivation est également écarté grâce à l'équité des niveaux de pression régnant au sein des conduits d'alimentation 10 et de dérivation 36.

## Revendications

1. Dispositif (1) d'irrigation et de mise en pression d'une cavité (2) organique, comprenant un circuit formé d'au moins une source (3), de laquelle s'écoule un fluide sous pression, connectée à un conduit d'alimentation (10) débouchant en son extrémité aval dans ladite cavité (2), un conduit d'évacuation (20) pour ce fluide ressortant de cette cavité (2) et des moyens de régulation (30) de ce fluide, **caractérisé en ce que** ces moyens de régulation (30) comprennent un premier moyen de pompage (31) pouvant être momentanément en prise avec le conduit d'alimentation (10), un second moyen de pompage (32) en prise avec le conduit d'évacuation (20) et **en ce que** ces deux moyens de pompage (31, 32) sont activés à la même vitesse et dans le même sens lorsqu'ils sont en prise avec leurs conduits respectifs (10, 20) .

2. Dispositif (1) selon la revendication 1, **caractérisé en ce que** lesdits moyens de régulation comprennent également un conduit de dérivation (36) mettant la cavité (2) sous pression, à la même valeur que celle dudit fluide.

3. Dispositif (1) selon la revendication 1, **caractérisé en ce qu'**une pompe péristaltique (35) à double conduits (10, 20) constitue à la fois le premier moyen de pompage (31) et le second moyen de pompage (32), et **en ce que** cette pompe est équipée d'au moins un moyen (33, 34) permettant de neutraliser l'effet de pompage sur au moins un desdits conduits (10, 20).

4. Dispositif (1) selon la revendication 3, **caractérisé en ce que** ledit moyen (33, 34) de neutralisation de l'effet de pompage est constitué par un sabot rétractable du conduit (10, 20) correspondant.

5. Dispositif (1) selon la revendication 1 ou 2, **caractérisé en ce que** le premier moyen de pompage (31) est constitué par une pompe péristaltique pourvue d'un moyen (33) permettant de neutraliser l'effet de pompage sur le conduit d'alimentation (10), le second moyen de pompage (32) est constitué par une seconde pompe péristaltique, et **en ce que** ces deux pompes sont reliées l'une à l'autre, soit mécaniquement, soit par un arbre électrique.

6. Dispositif (1) selon la revendication 1, **caractérisé en ce que** l'effet de pompage procuré par le second moyen de pompage (32) sur le conduit d'évacuation (20) peut être neutralisé par désaccouplement de ces deux derniers organes.

## Claims

1. A device (1) for irrigating and pressurizing an organ cavity (2), comprising a circuit formed of at least one source (3), from which there flows a pressurized fluid, connected to a supply pipe (10) opening at its downstream end into said cavity (2), a discharge pipe (20) for this fluid re-emerging from this cavity (2) and means (30) for regulating this fluid, **characterized in that** these regulating means (30) comprise a first pumping means (31) that can be temporarily engaged with the supply pipe (10), a second pumping means (32) engaged with the discharge pipe (20), and **in that** these two pumping means (31, 32) are activated at the same speed and in the same direction when they are engaged with their respective pipes (10, 20).

2. The device (1) as claimed in claim 1, **characterized in that** said regulating means also comprise a bypass pipe (36) placing the pressurized cavity (2) at the same pressure as the said fluid.

3. The device (1) as claimed in claim 1, **characterized in that** a peristaltic pump (35) with two pipes (10, 20) constitutes both the first pumping means (31) and the second pumping means (32), and **in that** this pump is equipped with at least one means (33, 34) able to neutralize the pumping effect on at least one of said pipes (10, 20).

4. The device (1) as claimed in claim 3, **characterized in that** said means (33, 34) of neutralizing the pumping effect consists of a retractable shoe of the corresponding pipe (10, 20).

5. The device (1) as claimed in claim 1 or 2, **characterized in that** the first pumping means (31) consists of a peristaltic pump provided with a means (33) of neutralizing the pumping effect on the supply pipe (10), the second pumping means (32) consists of a second peristaltic pump, and **in that** these two pumps are connected to one another either mechanically or by an electric shaft.

6. The device (1) as claimed in claim 1, **characterized in that** the pumping effect applied by the second pumping means (32) to the discharge pipe (20) can be neutralized by uncoupling the latter two components.

## Patentansprüche

1. Vorrichtung (1) zur Irrigation und Druckbeaufschlagung eines Organhohlraumes (2) mit einem Kreis, der aus zumindest einer Quelle (3), aus der ein unter Druck stehendes Fluid ausfliesst und die mit einem Zufuhrkanal (10) verbunden ist, der an seinem strömungsabwärts gelegenen Ende in den Hohlraum (2) mündet, einem Ableitungskanal (20) für dieses Fluid, der von diesem Hohlraum (2) wegführt, und Steuermitteln (30) für dieses Fluid gebildet wird, **dadurch gekennzeichnet, dass** diese Steuermittel (30) ein erstes Pumporgan (31), das zeitweise mit dem Zufuhrkanal (10) verbunden sein kann, sowie ein zweites Pumporgan (32) umfassen, das mit dem Ableitungskanal (20) verbunden ist, sowie **dadurch**, dass diese beiden Pumporgane (31, 32) mit der gleichen Geschwindigkeit und in der gleichen Richtung betrieben werden, wenn sie mit ihren jeweiligen Kanälen (10, 20) verbunden sind.

2. Vorrichtung (1) nach Anspruch 1, **dadurch gekennzeichnet, dass** die Steuermittel weiter eine Zweigleitung (36) umfassen, die den Hohlraum (2) mit dem gleichen Druck wie dem des Fluids beaufschlagen.

3. Vorrichtung (1) nach Anspruch 1, **dadurch gekennzeichnet, dass** eine Schlauchpumpe (35) mit zwei Kanälen (10, 20) zugleich das erste Pumporgan (31) und das zweite Pumporgan (32) darstellt, und **dadurch**, dass diese Pumpe mit zumindest einem Organ (33, 34) versehen ist, mit dem die Pumpwirkung auf zumindest einem der Kanäle (10, 20) neutralisiert werden kann.

4. Vorrichtung (1) nach Anspruch 3, **dadurch gekennzeichnet, dass** das Organ (33, 34) für die Neutralisierung der Pumpwirkung aus einem zurückziehbaren Gleitschuh des entsprechenden Kanals (10, 20) besteht.

5. Vorrichtung (1) nach Anspruch 1 oder 2, **dadurch gekennzeichnet, dass** das erste Pumporgan (31) aus einer Schlauchpumpe besteht, die mit einem Organ (33) versehen ist, mit dem die Pumpwirkung auf dem Zufuhrkanal (10) neutralisiert werden kann, während das zweite Pumporgan (32) aus einer zweiten Schlauchpumpe besteht, und **dadurch**, dass diese beiden Pumpen entweder mechanisch oder über eine elektrische Welle miteinander verbunden sind.

6. Vorrichtung (1) nach Anspruch 1, **dadurch gekennzeichnet, dass** die durch das zweite Pumporgan (32) auf den Ableitungskanal (20) ausgeübte Pumpwirkung durch Entkopplung dieser beiden letztgenannten Organe neutralisiert werden kann.
